# EUROPÄISCHE PATENTANMELDUNG

(11) **EP 1 585 050 A2**
(43) Veröffentlichungstag der Anmeldung: **12.10.2005**
(21) Anmeldenummer: 05090089.3
(22) Anmeldetag: 04.04.2005
(51) Int. Cl.: G06F 19/00

(54) **Verfahren zum Verwalten von medizinischen Bilddaten**

(30) Priorität: 05.04.2004 DE 102004017652
(71) Anmelder: Image Diagnost International GmbH, 81541 München (DE)
(72) Erfinder: Heinlein, Peter, Dr. rer. nat., 81549 München (DE); Dietrich, Sven, 80336 München (DE); Schneider, Wilfried, 85386 Eching (DE)
(74) Vertreter: Müller, Wolfram Hubertus

(57) **Zusammenfassung**

Die Erfindung betrifft ein Verfahren zum Verwalten medizinischer Bilddaten im DICOM-Format, bei dem mindestens ein Zugriffsmittel zum Zugriff auf die medizinischen Bilddaten vorgesehen ist. Erfindungsgemäß werden die medizinischen Bilddaten in einem Speichermittel (11) zentral gespeichert. Ein Regeler (10) regelt einen Zugriff auf die medizinischen Bilddaten, wobei der Zugriff auf die im Speichermittel (11) gespeicherten medizinischen Bilddaten von mindestens einem Zugriffsmittel (20) aus durch das Regler (10) gewährbar und unterbindbar ist.

## Beschreibung

Die Erfindung betrifft ein Verfahren zum Verwalten medizinischer Bilddaten nach Anspruch 1.

Als internationaler Standard zum Speichern medizinischer Daten und insbesondere medizinischer Bilddaten hat sich mittlerweile der DICOM-Standard durchgesetzt ("Digital Imaging and Communications in Medicine"). Der DICOM-Standard standardisiert die Datenstrukturen und Formate für medizinische Bilder und bildbezogene Daten und stellt somit ein Austauschformat zur Verfügung, dass das Abrufen von medizinischen Bilddaten durch mehrere Nutzer wie beispielsweise Ärzte und/oder medizinisches Fachpersonal von unterschiedlichen Orten aus ermöglicht.

Wird ein Patient von einem ersten Arzt in einer ersten Praxis zu einem zweiten Arzt einer zweiten Praxis überwiesen, kann der zweite Arzt die bereits in der ersten Praxis erstellten Daten des Patienten im DICOM-Format abrufen und in einem Speichersystem der zweiten Praxis abspeichern. Die Daten liegen dann in einem Format vor, das vom zweiten Arzt direkt weiter bearbeitet werden kann.

Der Erfindung liegt die Aufgabe zugrunde, die bislang bekannten Verwaltungsmethoden zur Speicherung von medizinischen Bilddaten zu verbessern.

Diese Aufgabe wird erfindungsgemäß durch ein Verfahren zum Verwalten medizinischer Bilddaten im DICOM-Format nach Anspruch 1 gelöst. Dabei werden die medizinischen Bilddaten zentral in einem Speichermittel gespeichert. Ein Regler regelt einen Zugriff auf die medizinischen Bilddaten. Dabei ist der Zugriff auf die medizinischen Bilddaten im Speichermittel von einem Zugriffsmittel aus durch den Regler gewährbar und unterbindbar.

Durch die zentrale Speicherung in einem einzigen Speichermittel ist es nicht mehr nötig, in mehreren Arztpraxen bzw. medizinischen Arbeitsgruppen die gleichen Bilddaten zu speichern. Mit den gleichen medizinischen Bilddaten in diversen Arztpraxen bzw. Arbeitsgruppen belegter Speicherplatz wird somit frei. Weiterhin kann es möglich bleiben, bei Bedarf Kopien der medizinischen Daten anzufertigen. Der Regler verwaltet Zugriffsrechte, durch die festgelegt wird, von welchen Zugriffsmitteln aus mit welchen Nutzerrechten auf das Speichermittel zugegriffen werden kann. Als solches Zugriffsmittel dient beispielsweise ein Computer.

In einer Ausführungsform wird durch den Regler geregelt, ob und welche der medizinischen Bilddaten durch das Zugriffsmittel abrufbar, veränderbar oder löschbar sind. Der Regler ist dabei so ausgebildet, dass es die Zugriffsrechte von unterschiedlichen Zugriffsmitteln individuell regelt. So können beispielsweise die Zugriffsrechte von Zugriffsmitteln von Ärzten so eingestellt sein, dass sie die medizinischen Bilddaten eines Patienten lesen und verändern können. Die Zugriffsrechte eines Zugriffsmittels des Patienten selber könnten gleichzeitig durch den Regler so geregelt werden, dass der Patient die Daten nur lesen kann.

Ein Vorteil der erfindungsgemäßen Lösung liegt in der Aktualität der zentralen Speicherung. Dadurch, dass Ärzte, die Zweit- oder Drittbefunde etc. für einen Patienten erstellen, über ein Zugriffsmittel direkt auf das zentrale Speichermittel zugreifen können, entstehen keine veralteten Kopien von Bilddaten eines Patienten, aus denen erst die aktuellste Fassung herauszusuchen ist. Die Ärzte speichern die Bilddaten direkt im zentralen Speichermittel ab und halten die Bilddaten des Patienten im Speichermittel aktuell, die von unterschiedlichen Zugriffsmitteln aus eingesehen werden können.

In einer besonders bevorzugten Ausführungsform erfolgt der Zugriff auf den medizinischen Bilddaten des Speichermittels durch das Zugriffsmittel über ein DICOM-Protokoll. Dies bedeutet, dass die medizinischen Bilddaten nicht nur im DICOM-Format aufnehmenden Zentralspeichermittel gespeichert sind, sondern auch in diesem Format über ein speziell für solche medizinische Bilddaten im DICOM-Format geeignetes Übertragungsprotokoll übertragen werden. Eine Umwandlung der Daten zum Übertragen ist also nicht notwendig, was einen beschleunigten Übertragungsprozess ermöglicht.

Bevorzugt werden im Speichermittel zusätzlich zu den medizinischen Daten auch medizinische Befunde, Diagnosen und Auswertungen als Daten gespeichert. Dabei sind die Daten und Bilddaten jeweils einem Patienten zugeordnet, so dass das Speichermittel als eine Art "Patientenaktenablage" dient, bei der über einen Patienten neben den Bildern auch beliebig viele andere Daten gespeichert werden können. Dabei weisen diese Daten vorzugsweise einen Bezug zu den gespeicherten Bilddaten auf, es handelt sich also beispielsweise um Diagnosen, die auf Grundlage bzw. mit Hilfe der medizinischen Bilddaten erstellt worden sind.

In einer besonders bevorzugten Ausführungsform gleicht das Speichermittel die auf ihm gespeicherten Daten (dabei sind Bilddaten und/oder übrige Daten gemeint) eines Patienten automatisch mit einer zentralen Patientendatei ab und aktualisiert die Daten. Als solche zentrale Patientendatei kann beispielsweise ein KIS (Krankenhausinformationssystem) oder ein RIS (Radiologieinformationssystem) dienen. In diesen Informationssystemen sind die Daten üblicherweise nur in Textform gespeichert, während Bilddaten in einem PACS (picture archiving and communication system) gespeichert werden.

Bevorzugt ist der Regler als Proxy-Server ausgebildet und regelt den Zugriff mindestens zweier Zugriffsmittel auf das zentrale Speichermittel anhand von Zugriffsrechten. Der Proxy-Server ist im zentralen Speichermittel vorgeschalten, so dass ein Zugriff auf das Speichermittel nur über den Proxy-Server möglich ist. Dieser prüft bei einem Zugriffswunsch durch ein Zugriffsmittel bzw. durch ein das Zugriffsmittel bedienendes Personal, ob das Zugriffsmittel überhaupt berechtigt ist, auf die Bilddaten zuzugreifen. Erst dann gewährt der Proxy-Server - in Abhängigkeit von der Prüfung - den eigentlichen Zugriff auf das zentrale Speichermittel oder auch nicht. Theoretisch kann ein Proxy-Server die Zugriffsrechte beliebig viele Zugriffsmittel regeln.

Ein Datenaustausch zwischen dem Zugriffsmittel und dem Regler (das als Verbindungsglied zum zentralen Speichermittel dient) erfolgt vorteilhaft über ein Komprimier- und Entkomprimiermittel. Dabei weist sowohl das Zugriffsmittel als auch der Regler ein Komprimier- und Entkomprimiermittel auf, das zu übertragene Daten vor und nach dem Übertragen komprimiert bzw. entkomprimiert. Gleiches gilt für die Übertragung von medizinischen Bilddaten zwischen zwei Zugriffsmitteln. Als solches Komprimier- und Entkomprimiermittel kann beispielsweise ein DICOM-Router dienen. Dadurch wird die zu übertragende Datenmenge reduziert und sowohl Übertragungskosten als auch Übertragungszeit eingespart. Bevorzugt werden die Bilddaten vom DICOM-Router zum Übertragen in ein JPEG 2000-Format oder anderes übliches komprimiertes Bildstab-Format umgewandelt.

In einer bevorzugten Ausführungsform werden in dem Speichermittel gespeicherte Daten und/oder Bilddaten über mindestens eines der Zugriffsmittel bearbeitet.

Dies bedeutet, dass nicht nur ein Übertragen der Bilddaten vom Speichermittel zum Zugriffsmittel möglich ist, sondern, dass mindestens ein Zugriffsmittel zugleich als ein Bedienungsmittel ausgebildet ist. Dadurch kann von diesem Zugriffsmittel aus auch direkt auf die im Speichermittel gespeicherten Daten zugegriffen und diese beispielsweise gelöscht werden. Das Zugriffsmittel dient also als ein Terminal, von dem aus - bei entsprechend dem Regler gespeicherten Zugriffsrechten - sich die gespeicherten Daten bearbeiten lassen.

Bevorzugt ist mindestens ein Digitalisierungsmittel vorgesehen, auf das über eines der Zugriffsmittel zugegriffen werden kann und die medizinischen Bilddaten digitalisiert werden können. Ein solches Digitalisierungsmittel kann entweder ein Aufnahmegerät sein, durch das digitale Bilder aufgenommen werden können, oder ein Umwandler, der analoge Bilder in digitale Bilder umwandelt. Die im Digitalisierungsmittel digitalisierten Bilder werden über den Regler an das Speichermittel zum Abspeichern übersandt. Dort werden sie im DICOM-Format abgespeichert. Daher dient das Digitalisierungsmittel insbesondere dazu, die Bilddaten sofort im DICOM-Format zu digitalisieren.

Bei dem Verfahren ist in einer Ausführungsform ein zentrales Detektionsmittel vorgesehen, durch das in den im Speichermittel gespeicherten medizinischen Bilddaten automatisch bestimmte pathologische Strukturen detektiert und vorgemerkt werden. Die vorgemerkten pathologischen Strukturen können im Anschluss von einem Arzt genauer untersucht und diagnostiziert werden. Dadurch, dass die Aufmerksamkeit des diagnostizierenden Arztes vom Detektionsmittel auf bestimmte pathologische Strukturen gelenkt wird, wird der Arzt bei der Diagnose durch das Detektionsmittel unterstützt. Das zentrale Detektionsmittel führt entweder auf Aufforderung des Personals oder je nach Ausführungsform beim Vorliegen neuer medizinischer Bilddaten automatisch einen erneuten Detektionsvorgang für die Bilder durch. Dabei ist das zentrale Detektionsmittel vornehmlich auf eine bestimmte Bilderart wie beispielsweise Mammographie-Aufnahmen spezialisiert.

Dabei kann vorteilhaft über ein Zugriffsmittel auf das zentrale Detektionsmittel zugegriffen werden und dadurch eine automatische Detektion pathologischer Strukturen ausgelöst oder bereits durchgeführte Detektionsergebnisse abgerufen werden. Die Detektionsergebnisse werden dabei bevorzugt mitsamt der daraufhin durch einen Arzt diagnostizierten Diagnosen im zentralen Speichermittel unter dem jeweiligen Patientenhinweis abgespeichert.

In einer besonders bevorzugten Ausführungsform wird ein Webserver verwendet, um den Regler mit dem Internet zu verbinden. So kann über das Internet beispielsweise mittels eines Webbrowsers über den Webserver auf den Regler und von dort auf das zentrale Speichermittel zugegriffen werden. In dieser Ausführungsform dient also jeder PC mit Internetzugang als Zugriffsmittel, von dem aus bei entsprechend in den Zugriffsrechten abgespeicherter Berechtigung auf die medizinischen Bilddaten zugegriffen werden kann.

Vorteilhaft werden die im Speichermittel gespeicherten Daten automatisch durch ein zentrales Planungsmittel ausgewertet. Das zentrale Planungsmittel plant künftige Handlungen zur Diagnose und/oder Behandlung von Patienten. Dabei kann es sich beispielsweise um die Vornahme von einer Zweit- oder Drittbefundung handeln, oder auch um einen Medikamenten-, Operations- oder Kurvorschlag für einen einzelnen Patienten. Das zentrale Planungsmittel kann auch automatisch feststellen, dass bei einem Patienten neue medizinische Bilddaten aufgenommen werden müssen. Bevorzugt regelt das Planungsmittel nach dem Erstellen einer Handlungsanweisung die Zugriffsrechte über den Regler dahingehend, dass Arztpraxen bzw. Arbeitsgruppen, die die Handlungsanweisungen betreffen (beispielsweise die neue medizinische Bilddaten aufnehmen sollen) automatisch Zugriffsrechte zum Lesen und Schreiben medizinischer Bilddaten von und auf dem zentralen Speichermittel zugewiesen werden.

Bevorzugt arbeiten an der Diagnose und/oder Behandlung eines Patienten mindestens zwei Arbeitsgruppen bzw. zwei Ärzte in unterschiedlichen Praxen, denen jeweils mindestens ein Zugriffsmittel zugeordnet ist. So kann über die mindestens zwei Zugriffsmittel auf dieselben medizinischen Bilddaten im zentralen Speichermittel zugegriffen werden. Die Daten können auch bei entsprechend vorhandenen Zugriffsrechten von den mindestens zwei Ärzten aktualisiert werden. Wird bei dem Verfahren wie oben beschrieben ein zentrales Planungsmittel verwendet, wird dabei die Arbeit der Arbeitsgruppen bevorzugt durch das Planungsmittel koordiniert. Dazu erstellt das Planungsmittel auf Grundlage der im Speichermittel abgespeicherten Daten automatisch Handlungsanweisungen für die Arbeitsgruppen zur Diagnose und/oder Behandlung eines Patienten. Diese sendet das Planungsmittel bevorzugt automatisch an die Zugriffsmittel der Arbeitsgruppen bzw. zumindest an das Zugriffsmittel einer betroffenen Arbeitsgruppe. Dadurch lassen sich die oft komplizierten Handlungsabläufe der zweiten und dritten Befundung bzw. des Aufnehmens neuer Bilddaten teilweise automatisieren und vereinfachen.

Bevorzugt wird mindestens eines der Zugriffsmittel als Befundungsmittel benutzt. Dies bedeutet, dass auf dem Zugriffsmittel zugleich die medizinischen Bilddaten dargestellt und von einem fachkundigen Personal begutachtet, also befundet wird. Dazu weist das Befundungsmittel vorzugsweise zwei Monitore auf, um eine große Anzeigefläche und eine gute Vergleichsmöglichkeit mehrerer Bilder bereitzustellen.

Bevorzugt werden dabei die medizinischen Bilddaten auf dem Befundungsmittel automatisch vorsortiert angezeigt, wobei Mammographieaufnahmen insbesondere nach mammographiespezifischen Kennzeichen wie Aufnahmewinkel oder Lateralität vorsortiert werden.

In einer bevorzugten Ausführungsform werden beim Aufruf einer Patientendatei von einem Zugriffsmittel aus automatisch Veränderungen speziell hervorgehoben angezeigt, die sich seit dem letzten Aufrufen der Patientendatei im zentralen Speichermittel ergeben haben. Solche Veränderungen können sich beispielsweise aufgrund des Zugriffs anderer Ärzte auf das zentrale Speichermittel ergeben haben.

Die Erfindung wird nachfolgend anhand von in Figuren dargestellten Ausführungsbeispielen näher erläutert. Es zeigen:
- Figur 1: ein Verfahren zum Verwalten von medizinischen Bilddaten im DICOM-Format, bei dem ein Regler den Zugriff auf ein zentrales Speichermittel regelt;
- Figur 2: das bisher bekannte Verfahren zum Verwalten von medizinischen Bilddaten im DICOM-Format; und
- Figur 3: die Vernetzung unterschiedlicher Einrichtungen zum Verwalten von medizinischen Bilddaten.

In den folgenden Figuren werden Verfahren zum Verwalten von medizinischen Bilddaten im DICOM-Format beschrieben. Dabei beziehen sich die Ausführungsbeispiele speziell auf ein Verfahren zum Verwalten von Mammographieaufnahmen. Das Verfahren kann aber auch ganz allgemein zum Verwalten von anderen medizinischen Bilddaten im DICOM-Format dienen.

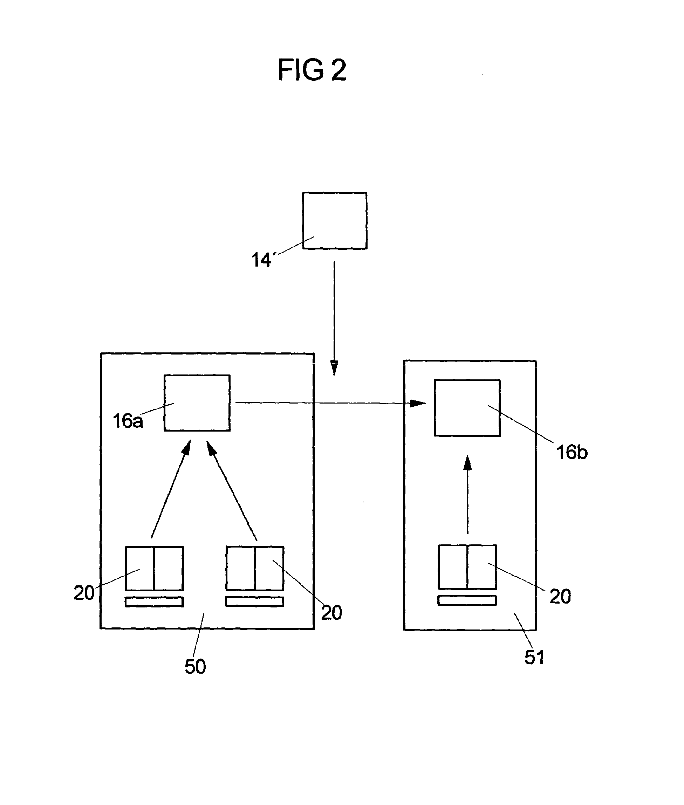

Die Figur 2 zeigt die bisherige Verwaltung von medizinischen Bilddaten im DICOM-Format. Eine erste Arbeitsgruppe 50 hat einen Patienten, von dem sie regelmäßig Aufnahmen macht, die digital im DICOM-Format im Archiv 16a gespeichert werden. Als Zugriffsmittel auf das Datenspeicherarchiv 16a dienen die beiden Workstations 20 der ersten Arbeitsgruppe 50. Sowohl das Datenspeicherarchiv 16a als auch die beiden Workstations 20 sind dabei der ersten Arbeitsgruppe 50 zugeordnet. Von den Workstations 20 aus können sämtliche Bilder im Archiv 16a bearbeitet, verändert, gelöscht, umbenannt etc. werden.

Ein Aufgabenserver 14' dient als Speichermittel zum Speichern diverser Aufgaben. Eine Aufgabe ist beispielsweise die Vornahme einer Zweitbefundung von medizinischen Bilddaten. Eine solche Zweitbefundung wird bei dem in Figur 2 dargestellten Verfahren durch eine zweite Arbeitsgruppe 51 vorgenommen. Der zweiten Arbeitsgruppe 51 ist ein Datenspeicher-Archiv 16b zugeordnet, auf das mittels einer Workstation 20' als Zugriffsmittel zugegriffen werden kann. Auch das Zugriffsmittel 20' ist der zweiten Arbeitsgruppe 51 zugeordnet.

Zur Durchführung einer zweiten Befundung ist eine "Überweisung" des Patienten vom Standort der ersten Arbeitsgruppe 50 zum Standort der zweiten Arbeitsgruppe 51 notwendig. Dazu werden die im Datenspeicher-Archiv 16a der ersten Arbeitsgruppe 50 gespeicherten medizinischen Bilddaten in das Datenspeicherarchiv 16b der zweiten Arbeitsgruppe 51 kopiert. Damit hat ein bedienendes Personal bzw. Arzt von der Workstation 20' der zweiten Arbeitsgruppe 51 aus vollen Zugriff auf die im Archiv 16b gespeicherten, "überwiesenen" medizinischen Bilddaten der ersten Arbeitsgruppe 50. Der Überweisungsvorgang kann ggf. von einem Aufgabenserver 14' gesteuert werden, der das Kopieren der Daten veranlasst.

Bei dem in Figur 2 gezeigten Verfahren werden die medizinischen Bilddaten dupliziert. Da beispielsweise Mammographieaufnahmen pro Patient eine Datenmenge von um die 160 MB in Anspruch nehmen, ergäbe dies in einem Screeningnetz eine duplizierte Datenmenge von mehreren Terra Byte.

Ein Zugriff auf eines der Datenspeicherarchive 16a, 16b oder eines anderen, nicht dargestellten Datenspeicherarchivs ist nur vom Standort einer Workstation aus realisierbar, die derselben Arbeitsgruppe wie das Archiv zugeordnet ist. Ein standortunabhängiger Zugriff ist nicht möglich. Deshalb ist z.B. der Zugriff von einer Workstation 20 der ersten Arbeitsgruppe 50 auf das Datenspeicherarchiv 16b der zweiten Arbeitsgruppe 51 unmöglich.

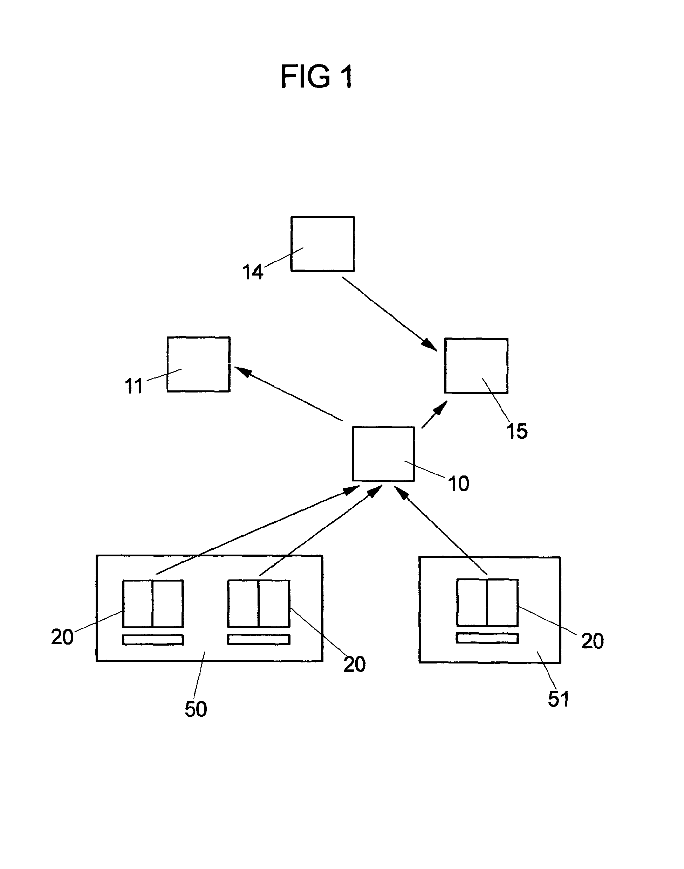

Die Figur 1 zeigt hingegen die Realisierung eines erfindungsgemäßen Verfahrens unter Zuhilfenahme eines zentralen Speichermittels 11, auf das der Zugriff von einem Proxy-Server 10 als Regler geregelt wird. Dazu werden Benutzerrechte 15 definiert, auf die der Proxy-Server 10 Zugriff hat und die definieren, von welchem Zugriffsmittel 20 bzw. 20' aus in welchem Umfang auf das zentrale Speichermittel 11 zugegriffen werden kann. Das zentrale Speichermittel 11 kann z.B. als PACS ausgebildet sein und aus einem beliebigen digitalen Speichermedium bestehen. Dabei ist sowohl eine Speicherung auf einem nicht wiederbeschreibbaren Medium wie beispielsweise einem Wechsler mit CD-ROM's möglich, als auch die Speicherung auf handelsüblichen Festplatten, Magnetbändern, Disketten oder Speicherbausteinen.

Die Zugriffsrechte sind individuell anpassbar und auf jede der Workstation 20 und 20' als Zugriffsmittel einzeln abstimmbar. So wird in den Zugriffsrechten 15 vermerkt, von welcher Workstation 20 aus bzw. mit welcher Kennung und mit welchem Login welche Daten im zentralen Speichermittel 11 gelesen, gelöscht, verändert oder neu erstellt werden dürfen. Somit sind grundsätzlich von jeder Workstation 20 aus die medizinischen Bilddaten eines Patienten im Speichermittel 11 einsehbar.

Figur 1 zeigt nun, dass von einer ersten Arbeitsgruppe 50 aus über zwei der Arbeitsgruppe 50 zugeordnete Workstations 20 und über den Proxy-Server auf das zentrale Speichermittel 11 als Datenarchiv zugegriffen werden kann und beispielsweise bei mammographischen Bildaufnahmen eine erste Befundung erstellt werden kann.

Dem zentralen Proxy-Server 10 sowie dem zentralen Speichermittel 11 ist ein zentrales Planungsmittel 14 als Aufgabenserver zugeordnet. Hat die erste Arbeitsgruppe 50 eine Erstbefundung zu den Mammographieaufnahmen eines Patienten erstellt, die im zentralen Speichermittel 11 gespeichert sind, erstellt das Planungsmittel 14 automatisch einen Plan zum weiteren Vorgehen: Zunächst wird automatisch eine Handlungsanweisung bzw. Aufgabe an eine zweite Arbeitsgruppe 51 gesandt, die zum Inhalt hat, dass durch die zweite Arbeitsgruppe 51 eine Zweitbefundung der Mammographieaufnahmen des Patienten durchgeführt werden soll. Zudem ändert das zentrale Planungsmittel 14 die Zugriffsrechte 15 der zweiten Arbeitsgruppe 51 bzw. der einen Workstation 20' der zweiten Arbeitsgruppe 51 dahingehend, dass die zweite Arbeitsgruppe 51 über den Proxy-Server 10 eben diese Bilddaten im zentralen Speichermittel 11 einsehen bzw. lesen kann.

Ein Fachpersonal der zweiten Arbeitsgruppe 51 kann nun von der Workstation 20' der zweiten Arbeitsgruppe 51 aus bequem die Bilddaten im zentralen Speichermittel 51 einsehen und eine Zweitbefundung erstellen.

Durch dieses Verfahren werden die Bilddaten nicht dupliziert oder noch weiter vervielfacht, sondern lediglich die Zugriffs- bzw. Benutzerrechte 15 geändert. Der Zugriff ist zudem unabhängig vom Standort, da nicht mehrere Archive mit teilweise unterschiedlichen oder nicht aktualisierten Daten genutzt werden, sondern ein zentrales, immer aktualisiertes Archiv im zentralen Datenspeichermittel 11.

Durch das in Figur 1 gezeigte Verfahren ist auch ein Gewähren von temporären Zugriffsrechten oder ein Entziehen von Zugriffsrechten für einzelne Arbeitsgruppen bzw. für einzelne Zugriffsmittel leicht realisierbar. Die Zugriffsrechte für einzelne Benutzer können beliebig komplex sein und auch innerhalb einer Arbeitsgruppe unterschiedlich zugeordnet sein.

Ein Zugriff kann entweder für jedes Zugriffsmittel, also jede Workstation 20 fest geregelt werden, oder über einen workstationunabhängigen Login erfolgen.

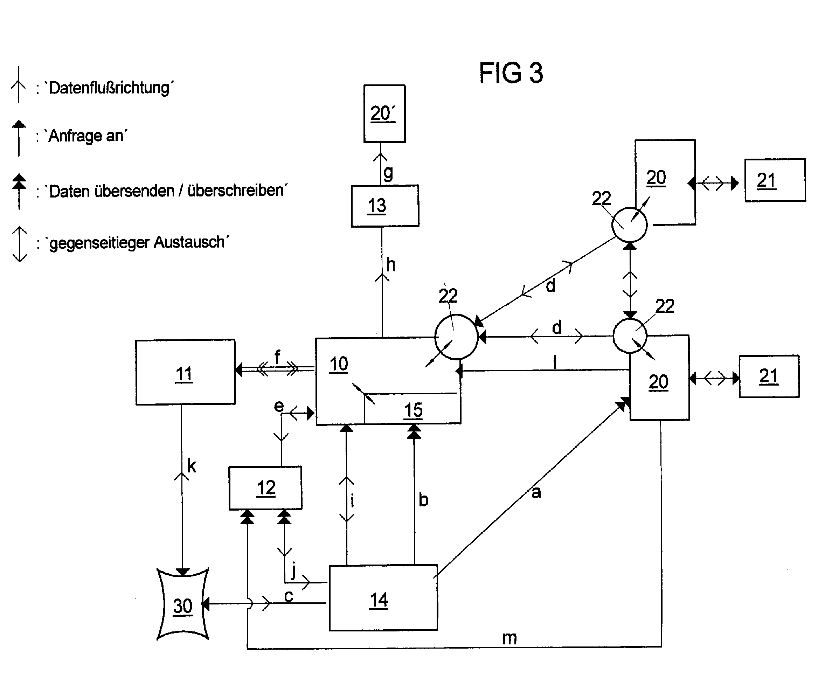

Eine andere Ausführungsform des erfindungsgemäßen Verfahrens zeigt die Figur 3. Hier wird ein Verfahren ausgenutzt, um die Kooperation mehrerer Standorte in einem Screeningverbund mit Zweitbefundung und einer übergeordneten Aufgabenverwaltung zu ermöglichen und zu verbessern. Dabei handelt es sich um einen Mammographie-Screeningverbund der von der Bildgebung über Erst- und Zweitbefundung sowie Abklärung im Assessment-Center alle Schritte übernimmt und überwacht. Eine reine Überweisung eines Patienten von einer Arbeitsgruppe in die nächste bzw. von einer Praxis in eine zweite (wie in Figur 2 gezeigt) ist dafür nicht ausreichend.

Figur 3 zeigt ein Konzept zum Einsatz standardisierter Komponenten zu einem speziellen System, dass das erfindungsgemäße Verfahren zum Aufbau eines digitalen Arbeitsablaufs für Screeningnetze ausnutzt. Dabei werden Bilddaten verschiedener Krankenhäuser in dem zentralen Archiv 11 als Speichermittel gespeichert. Für eine flexible Zugriffsbeschränkung sorgt der Proxy-Server 10, der die einzige Möglichkeit eines Zugriffs auf das zentrale Archiv 11 bereitstellt. Als Planungsmittel dient der Aufgabenserver 14, der sowohl die für die Teilnehmer anstehenden Optionen (Befundung, Zweitbefundung etc.) koordiniert, als auch die Zugriffsrechte 15 automatisch verwaltet, auf die der Proxy-Server 10 zurückgreift, um den Zugriff von einem Zugriffsmittel 20/20' aus auf das zentrale Speicherarchiv 11 zu überprüfen.

Wesentlich dabei ist das Planungsmittel 14, das hier als "Worklist-" und "Kollaborationsserver" ausgebildet ist. Das Planungsmittel 14 überprüft den Stand der Befundung eines Patienten, die Vollständigkeit der gesammelten Daten bzw. Bilddaten, überprüft Nachfragen, Indikationsstellung, dient zur Erfassung von Vorbefunden, die auf Grundlage vorbereiteter Fragebögen erstellt wurden und den Stand der Zweitbefundung. Für diese Vielzahl von Aufgaben stellt das Planungsmittel 14 eine exakte Statusverwaltung zur Verfügung, die einzelne Arbeitsgruppen automatisch an noch nicht erledigte Arbeiten erinnert und bei abgeschlossener Befundung oder Diagnostik die Daten automatisch über den Regler 10 an das zentrale Speichermittel 11 überträgt. Das Planungsmittel 14 kann auch zum sogenannten "Patiententracking" dienen. Der Server des Planungsmittels stellt Arbeitslisten für alle Workstations 20 und Digitalisierungsstation 21 bereit.

An den Workstations 20 arbeitet jeweils ein Arzt oder Fachpersonal, das die Workstation zur Befundung nutzt, indem sie dort medizinische Bilddaten aus dem zentralen Speichermittel 11 analysiert, und/oder das Digitalisierungsmittel 21 zur Aufnahme und/oder Digitalisierung weiterer medizinischer Bilddaten verwendet. Wie durch Pfeil a dargestellt, übersendet das Planungsmittel 14 einen Auftrag an eine Workstation 20 als Zugriffsmittel, beispielsweise den Auftrag eine Zweitbefundung durchzuführen. Gleichzeitig ändert das Planungsmittel 14 die Zugriffsrechte 15 dahingehend, dass der Zweitbefunder an der Workstation 20 neue Bilddaten für einen bestimmten Patienten im zentralen Speichermittel 11 abspeichern darf (Pfeil b).

Das Planungsmittel 14 ermöglicht es, standortübergreifende Arbeitsablaufszenarien einzurichten. Weiterhin übernimmt es unvollständige Patientenstammdaten aus der zentralen Patientendatenbank 30, die hier als RIS (Radiologieinformationssystem) ausgebildet ist (Pfeil c). Durch das Planungsmittel 14 als Kollaborationsserver ist die Zugriffsverwaltung entsprechend den Datenschutzbestimmungen gewährleistet, da das Auslesen des Archivbestandes abhängig von den Benutzerrechten des Anwenders einschränkbar ist.

Bei der Bildaufnahme in den einzelnen Arbeitsgruppen bzw. von den einzelnen Ärzten wird u.a. die Speicherfolientechnik und die direkt digitale Mammographie (FFDM) zur Monitorbefundung ausgenutzt. Wie verschiedene Studien ergeben haben, reduziert die digitale Archivierung der Bilddaten die Fixkosten erheblich. Dadurch sind Voraufnahmen "auf Knopfdruck" verfügbar. Aufnahmen werden durch die Mammographie-Workstations 20 automatisch vorsortiert angezeigt, wobei ein zeitraubendes, manuelles Aufhängen von Aufnahmen an einen Lichtkasten entfällt. Die Workstations 20 eignen sich sowohl für die digitalisierte Filmmammographie als auch für Speicherfolientechnik oder direkt für digitale Mammographie. Eine spezielle Software dient zur Integration von Ultraschallbildern, MR-Mammographien und histologischen Aufnahmen zur Befundung.

Da die einzelnen Workstations 20 als Zugriffsmittel räumlich oft weit voneinander getrennt sind (z.B. in anderen Krankenhausflügeln oder anderen Krankenhäusern), besteht die Notwendigkeit für eine digitale Bildübertragung in einer erforderlichen Qualität, die wirtschaftlich vertretbar ist.

Für eine Doppelbefundung ist es oft notwendig, eine zweite Expertenmeinung aus einem räumlich entfernten Kompetenzzentrum mit einem anderen Zugriffsmittel 20 einzuholen (Telemammographie). Deswegen werden die Daten zur Übertragung zwischen dem Proxy-Server 10 und der Workstation 20 von einem DICOM-Router 22 komprimiert und beim Empfangen wieder entkomprimiert (Pfeile d). Auch bei einem Datentransfer zwischen zwei Workstations 20 können die Daten über einen DICOM-Router 22 komprimiert und entkomprimiert werden (Pfeil d').

Zahlreiche internationale Studien haben ergeben, dass Verfahren zur computerassistierten Diagnose (CAD) eine hohe Befundungsqualität sichern und zur frühen Erkennung von Tumoren auf Mammographieaufnahmen beitragen können. Insbesondere zur Detektion von Mikroverkalkungen und Verdichtungen ist ein solcher CAD-Server nützlich. Er detektiert und markiert automatisch Tumormerkmale, wobei er Algorithmen verwendet, die auf Wavelettransformationen basieren und zur Kontrastverstärkung dienen. Ein solcher CAD-Server ist in Figur 3 als Detektionsmittel 12 gezeigt. Der CAD-Server 12 hat über den Proxy-Server 10 Zugriff auf den Bilddatenbestand des zentralen Speichermittels 11 (Pfeile e und f).

Der Proxy-Server 10 ist weiterhin mit einem Webserver 13 verbunden, über den ein Nutzer wie beispielsweise ein Patient von einem Zugriffsmittel 20' aus Zugriffsrechte auf Teile der im zentralen Speichermittel 11 gespeicherten Daten gewährt werden können. Vom Zugriffsmittel 20' aus kann sich ein Nutzer beispielsweise mit einem beliebigen Internetbrowser auf dem Webserver 13 einloggen (Pfeil g), welcher am Proxy-Server 10 die entsprechenden Bilddaten abfragt (Pfeil h). Der Proxy-Server 10 schaut daraufhin in den Zugriffsrechten 15 nach, ob vom Zugriffsmittel 20' aus die abgefragten Daten im Speichermittel 11 abgefragt werden dürfen. Erlauben die Zugriffsrechte 15 einen solchen Zugriff, leitet der Proxy-Server 10 die Abfrage an das zentrale Speichermittel 11 weiter (Pfeil f). Die Daten werden dann wieder über den Proxy-Server 10 und den Webserver 13 an das Zugriffsmittel 20' zurückgesandt.

Vom Planungsmittel 14 aus kann über den Proxy-Server 10 (Pfeil i) auf das zentrale Speichermittel 11 zugegriffen werden (Pfeil f), um die medizinischen Bilddaten weiter zu bearbeiten. Dabei werden u.a. automatisch Detektionsergebnisse des Detektionsmittels 12 für neu erstellte Bilddaten angefordert (Pfeil j).

Das zentrale Mammographiearchiv 11 speichert medizinische Bilddaten, Befunde, Ergebnisse des CAD-Servers 12 und weitere Daten von angeschlossenen Teilnehmern. Es dient zur Langzeitarchivierung als PACS-Archiv der Teilnehmer und kann wie das Planungsmittel 14 seine Daten mit den Daten an einer zentralen Krankenhausdatei 30 abgleichen (Pfeil k).

Die Verbindung zwischen den Workstations 20 und dem Proxy-Server 10 besteht nicht allein über die DICOM-Router 22, sondern kann auch direkt ausgebildet sein (Pfeil I). Je nach Zugriffsrechten kann von der Workstation 20 aus auch ein erneuter automatischer Detektionsvorgang durch den CAD-Servers 12 angefordert werden. Beim Vorhandensein von Administratorrechten können die Zugriffsrechte 15 manuell verändert werden.

Die in Figur 3 gezeigten Pfeile zur Veranschaulichung der Verbindungsarten zwischen den unterschiedlichen Komponenten sind dabei beispielhaft zu verstehen. Es versteht sich dabei von selbst, dass Verbindungen üblicherweise in beide vorhandene Richtungen erstellt werden können. Die Pfeile symbolisieren nur die vornehmlich gestellten Anfragen bzw. Datenflüsse und sind nicht explizit zu verstehen.

### Bezugszeichenliste

- 10: Regler
- 11: Speichermittel
- 12: Detektionsmittel
- 13: Web-Server
- 14: Planungsmittel
- 15: Zugriffsrechte
- 16, 16a, 16b: Bilddatenspeicher einzelner Gruppen
- 20,20': Zugriffsmittel
- 21: Digitalisierungsmittel
- 22: Komprimier- und Entkomprimiermittel
- 30: zentrale Patientendatei
- 40: Internet
- 50: erste Arbeitsgruppe
- 51: zweite Arbeitsgruppe

## Patentansprüche

1. Verfahren zum Verwalten medizinischer Bilddaten im DICOM-Format, bei dem mindestens ein Zugriffsmittel zum Zugriff auf die medizinischen Bilddaten vorgesehen ist, wobei
- die medizinischen Bilddaten in einem Speichermittel (11) zentral gespei- chert werden;
- ein Regler (10) den Zugriff auf die medizinischen Bilddaten regelt; und
- der Zugriff auf die im Speichermittel (11) gespeicherten medizinischen Bilddaten von dem mindestens einen Zugriffsmittel (20) aus durch den Regler (10) gewährt oder unterbunden wird.

2. Verfahren nach Anspruch 1, **dadurch gekennzeichnet, dass** durch den Regler (10) geregelt wird, ob und welche medizinische Bilddaten durch das Zugriffsmittel (20) abrufbar, veränderbar und/oder löschbar sind.

3. Verfahren nach Anspruch 1 oder 2, **dadurch gekennzeichnet, dass** der Zugriff auf die im Speichermittel (11) gespeicherten medizinischen Bilddaten in Abhängigkeit von Zugriffsrechten gewährt oder unterbunden wird.

4. Verfahren nach einem der vorangegangenen Ansprüche, **dadurch gekennzeichnet, dass** ein Zugriff auf die medizinischen Bilddaten des Speichermittels (11) durch das Zugriffsmittel (20) über ein DICOM-Protokoll erfolgt.

5. Verfahren nach einem der vorangegangenen Ansprüche, **dadurch gekennzeichnet, dass** im Speichermittel (11) zusätzlich medizinische Befunde, Diagnosen und Auswertungen als Daten gespeichert werden.

6. Verfahren nach einem der vorangegangenen Ansprüche, **dadurch gekennzeichnet, dass** die im Speichermittel (11) gespeicherten Daten und Bilddaten jeweils einem Patienten zugeordnet sind.

7. Verfahren nach Anspruch 6, **dadurch gekennzeichnet, dass** das Speichermittel (11) die gespeicherten Daten eines Patienten automatisch mit einer zentralen Patientendatei (30) abgleicht und aktualisiert.

8. Verfahren nach einem der vorangegangenen Ansprüche, **dadurch gekennzeichnet, dass** der Regler (10) als Proxy-Server ausgebildet ist und den Zugriff mindestens zweier Zugriffsmittel (20) anhand von Zugriffsrechten regelt.

9. Verfahren nach einem der vorangegangenen Ansprüche, **dadurch gekennzeichnet, dass** ein Datenaustausch zwischen dem Zugriffsmittel (20) und dem Regler (10) und/oder zwischen zwei Zugriffsmitteln (20) über ein Komprimier- u. Entkomprimiermittel (22) erfolgt.

10. Verfahren nach Anspruch 9, **dadurch gekennzeichnet, dass** das Komprimier- u. Entkomprimiermittel (22) als Router ausgebildet ist.

11. Verfahren nach einem der vorangegangenen Ansprüche, **dadurch gekennzeichnet, dass** über das Zugriffsmittel (20) in dem Speichermittel (11) gespeicherte Daten und / oder Bilddaten bearbeitet werden.

12. Verfahren nach einem der vorangegangenen Ansprüche, **dadurch gekennzeichnet, dass** über das Zugriffsmittel (20) und mittels eines Digitalisierungsmittels (21) medizinische Bilddaten digitalisiert und über das Regler (10) an das Speichermittel (11) zum Abspeichern übersandt werden.

13. Verfahren nach einem der vorangegangenen Ansprüche, **dadurch gekennzeichnet, dass** durch ein zentrales Detektionsmittel (12) die im Speichermittel (11) gespeicherten medizinischen Bilddaten automatisch auf bestimmte pathologische Strukturen hin untersucht werden.

14. Verfahren nach Anspruch 13, **dadurch gekennzeichnet, dass** über das Zugriffsmittel (20) auf das zentrale Detektionsmittel (12) zugegriffen wird.

15. Verfahren nach einem der vorangegangenen Ansprüche, **dadurch gekennzeichnet, dass** der Regler (10) über einen Web-Server (13) eine Internetverbindung bereitstellt, durch die über das Internet (40), den Web-Server (13) und das Regler (10) auf das Speichermittel (11) zugegriffen werden kann.

16. Verfahren nach einem der vorangegangenen Ansprüche, **dadurch gekennzeichnet, dass** durch ein zentrales Planungsmittel (14) die im Speichermittel (11) gespeicherten Daten automatisch dahingehend ausgewertet werden, künftige Handlungen zur Diagnose und/oder Behandlung von Patienten zu planen.

17. Verfahren nach mindestens Anspruch 16, **dadurch gekennzeichnet, dass** das Planungsmittel (14) automatisch Zugriffsrechte regelt, auf deren Grundlage der Regler (10) den Zugriff auf die medizinischen Bilddaten im zentralen Speichermittel (11) regelt.

18. Verfahren nach einem der vorangegangenen Ansprüche, **dadurch gekennzeichnet, dass** an der Diagnose und/oder Behandlung eines Patienten mindestens zwei Arbeitsgruppen (50; 51) arbeiten, denen jeweils mindestens ein Zugriffsmittel (20) zugeordnet ist.

19. Verfahren nach Anspruch 16 oder 17 und Anspruch 18, **dadurch gekennzeichnet, dass** die Arbeit der Arbeitsgruppen (50; 51) durch das Planungsmittel (14) koordiniert wird, indem das Planungsmittel (14) auf Grundlage der im Speichermittel (11) abgespeicherten Daten automatisch Handlungsanweisungen für die Arbeitsgruppen (50; 51) zur Diagnose und/oder Behandlung eines Patienten erstellt.

20. Verfahren nach Anspruch 19, **dadurch gekennzeichnet, dass** das Planungsmittel (14) automatisch Handlungsanweisungen zur Diagnose und/oder Behandlung eines Patienten an die Zugriffsmittel (20) der Arbeitsgruppen (50; 51) sendet.

21. Verfahren nach einem der vorangegangenen Ansprüche, **dadurch gekennzeichnet, dass** in dem Speichermittel (11) Mammographiedaten als medizinische Bilddaten gespeichert werden.

22. Verfahren nach einem der vorangegangenen Ansprüche, **dadurch gekennzeichnet, dass** mindestens ein Zugriffsmittel (20) als Befundungsmittel benutzt wird.

23. Verfahren nach Anspruch 22, **dadurch gekennzeichnet, dass** auf dem Befundungsmittel die medizinischen Bilddaten aus dem Speichermittel (11) auf zwei Monitoren dargestellt werden.

24. Verfahren nach Anspruch 21 und Anspruch 22 oder 23, **dadurch gekennzeichnet, dass** die medizinischen Bilddaten auf dem Befundungsmittel je nach ihren mammographiespezifischen Kennzeichen automatisch vorsortiert angezeigt werden.

25. Verfahren nach einem der Ansprüche 22 bis 24, **dadurch gekennzeichnet, dass** auf dem Befundungsmittel bei Aufruf eines Patienten automatisch Veränderungen angezeigt werden, die sich seit der letzten Bearbeitung des Patienten mit dem Befundungsmittel in den zu dem Patienten im zentralen Speichermittel (11) gespeicherten Daten und/oder Bilddaten ergeben haben.
